(19) Europäisches Patentamt
European Patent Office
Office européen des brevets

(11) Veröffentlichungsnummer: **0 158 921**
**A2**

# (12) EUROPÄISCHE PATENTANMELDUNG

(21) Anmeldenummer: 85103950.3

(22) Anmeldetag: 02.04.85

(51) Int.Cl.$^4$: **C 07 D 233/88**
//A01N43/50

(30) Priorität: 14.04.84 DE 3414115

(43) Veröffentlichungstag der Anmeldung: 23.10.85 Patentblatt 85/43

(84) Benannte Vertragsstaaten: CH DE FR GB IT LI

(71) Anmelder: BAYER AG
Konzernverwaltung RP Patentabteilung
D-5090 Leverkusen 1 Bayerwerk(DE)

(72) Erfinder: Buschhaus, Hans-Ulrich, Dr.
Morgengraben 2
D-5000 Köln 80(DE)

(72) Erfinder: Findeisen, Kurt, Dr.
In der Follmühle 10
D-5068 Odenthal(DE)

## (54) Verfahren zur Herstellung von N'N'-disubstituierten 5-Imino-imidazolidindionen und neue 5-Imino-imidazollidindion-Derivate.

(57) N,N'-disubstituierte 5-Imino-imidazolidindione werden nach einem neuen Verfahren durch Aminolyse von N,N'-disubstituierten 5-Acylimino-imidazolidindionen mit primären Aminen hergestellt. Nach dem Verfahren werden auch neue 5-Imino-imidazolidindion- Derivate mit fungiziden und herbiziden Eigenschaften erhalten.

EP 0 158 921 A2

0158921

BAYER AKTIENGESELLSCHAFT 5090 Leverkusen, Bayerwerk
Konzernverwaltung RP B/by-c
Patentabteilung

# Verfahren zur Herstellung von N,N'-disubstituierten 5-Imino-imidazolidindionen und neue 5-Imino-imidazolidindion-Derivate

Die Erfindung betrifft ein neues Verfahren zur Herstellung von N,N'-disubstituierten 5-Imino-imidazolidindionen.

N,N'-disubstituierte 5-Imino-imidazolidindione, bei denen die Iminogruppe in 5-Stellung unsubstituiert ist, sind aus Chem. Ber. 38, 2977 (1905) bekannt. Die Verbindungen werden durch Umsetzung eines Isocyanats mit Blausäure hergestellt.

N,N'-disubstituierte 5-Imino-imidazolidindione, bei denen die Iminogruppe in 5-Stellung durch Alkyl- oder Aryl-Gruppen substituiert ist, sind aus der SU-PS 529 160 bekannt. Sie werden durch Umsetzung von N,N'-disubstituierten 5-Alkylhydroxylamino-imidazolidindionen mit Isocyanat in Gegenwart von Triethylamin erhalten.

Aus den US-PS 39 25 553 und 39 92 402 ist bekannt, 5-Polyhaloethylimino-2,4-imidazolidindione durch Umsetzung von 5-Imino-2,4-imidazolidindionen mit Chloral herzustellen.

Nachteilig an diesen bekannten Verfahren ist, daß sie die Verwendung instabiler Ausgangsverbindungen erfordern und daß deshalb die Ausführung dieser Verfahren in technischem Maßstab große Schwierigkeiten bereitet, wenn nicht gar unmöglich ist.

Es wurde nun gefunden, daß sich 5-Imino-imidazolidindione auf einfache Weise ausgehend von stabilen, gut lager- und handhabbaren Ausgangsverbindungen, nämlich durch Aminolyse von 5-Acylimino-imidazolidindionen mit primären Aminen herstellen lassen.

Die Erfindung betrifft daher ein neues Verfahren zur Herstellung von N,N'-disubstituierten 5-Imino-imidazolidindionen, das dadurch gekennzeichnet ist, daß man aus N,N'-disubstituierten 5-Acylimino-imidazolidindionen den Acylrest durch Aminolyse mit primären Aminen abspaltet.

Bei den erfindungsgemäß herzustellenden N,N'-disubstituierten 5-Imino-imidazolidindionen handelt es sich vorzugsweise um Verbindungen der Formel

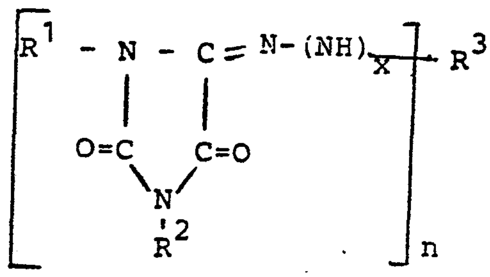

(I)

in der

n 1, 2 oder 3 bedeutet,

x 0 oder 1 ist,

$R^1$ und $R^2$ unabhängig voneinander für einen gegebenenfalls substituierten aliphatischen, cycloaliphatischen, araliphatischen, aromatischen oder heteroaromatischen Rest stehen und

$R^3$ einen n-wertigen, gegebenenfalls substituierten aliphatischen, cycloaliphatischen, aromatischen oder heteroaromatischen Kohlenwasserstoff-Rest bedeutet.

Als Ausgangsverbindungen dienen vorzugsweise 5-Acylimino-imidazolidindione der Formel

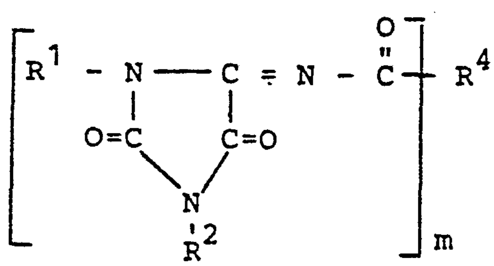

(II)

in der

m 1, 2 oder 3 bedeutet,

$R^1$ und $R^2$ die unter Formel (I) angegebene Bedeutung haben, und

$R^4$ für einen m-wertigen gegebenenfalls substituierten aliphatischen, cycloaliphatischen, araliphatischen oder aromatischen Rest steht.

Die zur Aminolyse der 5-Acylimino-imidazolidindione der Formel (II) zu verwendenden primären Amine entsprechen vorzugsweise der Formel

$$[H_2N-(NH)_x]_n R^3 \qquad (III)$$

in der

n, x und $R^3$ die unter Formel (I) angegebene Bedeutung haben.

Das erfindungsgemäße Verfahren kann durch folgende Reaktionsgleichung erläutert werden:

Der Verlauf der Aminolyse ist insofern überraschend, als zu erwarten war, daß bei der Behandlung der Imidazolidindione mit Aminen eine Öffnung des Imidazolin-Ringes eintritt.

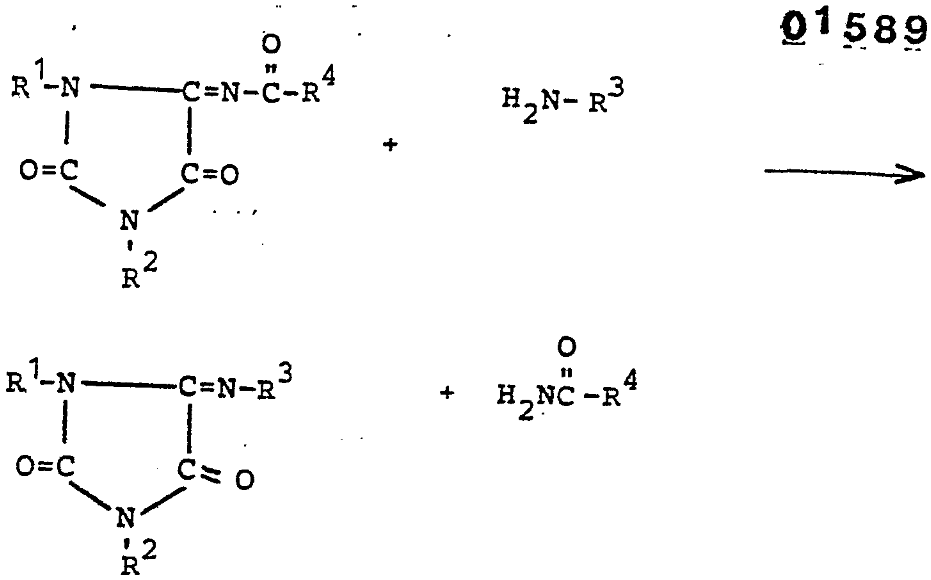

Der Verlauf der erfindungsgemäßen Aminolyse ist von der Natur der Substituenten $R^1$, $R^2$, $R^3$ und $R^4$ unabhängig. Vorteilhaft wählt man als Acylrest

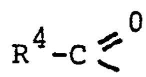

einen billigen, technisch leicht zugänglichen Rest, der möglichst außerdem noch ein Säureamid

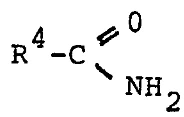

bildet, das seinerseits technisch verwertbar ist, so daß bei der Aminolyse kein Abfallprodukt entsteht.

Nach dem erfindungsgemäßen Verfahren erhält man die N,N'-disubstituierten 5-Imino-imidazolidindione in hohen Ausbeuten. Das erfindungsgemäße Verfahren läßt sich einfach durchführen und eignet sich daher auch besonders für die Herstellung dieser Verbindungen in technischem Maßstab.

Im Rahmen der vorliegenden Erfindung haben die in den Definitionen der Reste $R^1$, $R^2$, $R^3$ und $R^4$ verwendeten Begriffe aliphatischer, cycloaliphatischer, araliphatischer, aromatischer und heteroaromatischer Kohlenwasserstoffrest folgende Bedeutung:

0158921

Unter aliphatischen Kohlenwasserstoffresten sind geradkettige oder verzweigte $C_1$-$C_{20}$-, vorzugsweise $C_1$-$C_{12}$- und ganz besonders bevorzugt $C_1$-$C_6$-Alkyl-Reste zu verstehen; beispielsweise seien genannt: Methyl, Ethyl, Propyl, Isopropyl, Butyl, Isobutyl, Pentyl, Isopentyl, Hexyl und Isohexyl.

Unter cycloaliphatischen Resten sind Cycloalkylreste mit 3 bis 8, vorzugsweise 4, 5 oder 6 Kohlenstoffatomen zu verstehen; beispielsweise seien genannt Cyclopropyl, Cyclobutyl, Cyclopentyl, Cyclohexyl, Cycloheptyl und Cyclooctyl. Bevorzugt sind Cyclopentyl und Cyclohexyl.

Unter araliphatischen Resten sind durch Phenylreste substituierte Alkylreste z.B. der Benzyl-, 2-Phenylethyl-, der 3-Phenylpropyl- und der 2-Naphthyl-ethylrest zu verstehen.

Unter aromatischen Resten werden aromatische Kohlenwasserstoffe mit 6 bis 12 Kohlenstoffatomen verstanden; beispielsweise seien genannt: Phenyl, Naphthyl, Diphenyl.

Unter heteroaromatischen Resten sind 5- oder 6-gliedrige Ringe, die außer Kohlenstoffatomen noch 1 bis 3 Heteroatome, vorzugsweise ein Heteroatom, enthalten, zu verstehen. Heteroatome sind vorzugsweise Stickstoff, Schwefel und Sauerstoff, vorzugsweise Stickstoff. Beispielsweise seien genannt: Der Pyridinyl-(2)-, Pyridinyl-(4)-, Pyrimidinyl-(2)-, 4-Methyl-pyrimidinyl-(2)-, 6-Phenyl-1,3,5-triazinyl-(2)- oder -(4)- und 1,3,5-Triazinyl-(2)- oder -(4)-Rest.

Die Reste $R^1$, $R^2$ und $R^4$ können ihrerseits durch solche Atome oder Atomgruppen substituiert sein, die sich unter

den angewendeten Reaktionsbedingungen nicht verändern. Als solche unter den Reaktionsbedingungen inerte Substituenten seien beispielsweise genannt: Hydroxyl, $C_1$-$C_6$-Alkyl, $C_1$-$C_6$-Alkylamino, Di-($C_1$-$C_6$alkyl)-amino, Halogen wie Fluor, Chlor, Brom oder Iod, vorzugsweise Chlor, Nitro, Carbonyl, Carbonyloxy, Thiocarbonyl.

Der Rest $R^3$ kann durch Hydroxyl, $C_1$-$C_6$-Alkyl, $C_1$-$C_6$-Alkylamino, Di-($C_1$-$C_6$-alkyl)-amino, Halogen wie Fluor, Chlor, Brom und Iod, vorzugsweise Chlor, -$SO_3H$ oder $C_6$-$C_{15}$-Aryl, vorzugsweise Phenyl oder Carbonyloxy substituiert sein. Bevorzugte Substituenten des Restes $R^3$ sind: $C_1$-$C_6$-Alkyl, Di-($C_1$-$C_6$-alkyl)-amino und Carbonyloxy.

Bevorzugt verwendete 5-Acylimino-imidazolidindione sind solche Verbindungen der Formel (II) in der $R^1$ und $R^2$ unabhängig voneinander einen gegebenenfalls durch Hydroxyl, $C_1$-$C_6$-Alkyl, durch Chlor oder Phenyl substituiertes $C_1$-$C_{12}$-Alkyl oder $C_5$-$C_6$-Cycloalkyl, Phenyl oder Benzyl bedeuten und $R^4$ für einen n-wertigen, gegebenenfalls durch Hydroxyl, $C_1$-$C_6$-Alkyl, Chlor oder Phenyl substituierten $C_1$-$C_{20}$-Alkyl-, $C_5$-$C_6$-Cycloalkyl-, Phenyl- oder Benzylrest steht.

Die Herstellung der im erfindungsgemäßen Verfahren als Ausgangsverbindungen zu verwendenden 5-Acylimino-imidazolidindione ist bekannt (siehe DE-OS 32 11 911). Sie können beispielsweise hergestellt werden, indem man die an sich bekannten 5-Trialkylsilyliminoimidazolidindione (vgl. Inanda. I. Ojima; J. Organomet Chem. 169, (1979), 171) mit Carbonsäurehalogeniden oder Carbonsäureanhydriden umsetzt.

Als Beispiele für die erfindungsgemäß als Ausgangsverbindungen zu verwendenden 5-Acylimino-imidazolidindione seien genannt: N,N'-Dimethyl-5-acetylimino-imidazolidindion, N,N'-Dibutyl-5-acetylimino-imidazolidindion, N,N'-Distearyl-5-acetylimino-imidazolidindion, N,N'-Dimethyl-5-benzoylimino-imidazolidindion, N,N'-Dibutyl-5-benzoylimino-imidazolidindion, das Umsetzungsprodukt von 2 Mol N,N'-Dibutyl-5-trimethylsilylimino-imidazolidindion mit einem Mol Bernsteinsäuredichlorid, das Umsetzungsprodukt von 2 Mol N,N'-Dibutyl-5-trimethylsilyl-imino-imidazolidindion mit einem Mol Adipinsäuredichlorid, das Umsetzungsprodukt von 2 Mol N,N'-Dimethyl-5-trimethylsilyl-imino-imidazolidindion mit einem Mol Terephthalsäuredichlorid und das Umsetzungsprodukt von 3 Mol N,N'-Dimethyl-5-trimethylsilyl-imino-imidazolidindion mit einem Mol 1,3,5-Benzoltricarbonsäuretrichlorid.

Zur erfindungsgemäßen Aminolyse werden vorzugsweise solche Amine der Formel (III) verwendet, in der $R^3$ für einen n-wertigen, 1 bis 10 C-Atome aufweisenden aliphatischen, einen 5 oder 6 C-Atome aufweisenden cycloaliphatischen oder einen 6 bis 12 C-Atome aufweisenden aromatischen Kohlenwasserstoffrest steht.

Die Reste können durch Hydroxyl, $C_1$-$C_6$-Alkyl, Chlor oder Phenyl substituiert sein.

Die für die erfindungsgemäße Aminolyse zu verwendenden primären Amine können sowohl Mono-, Di- als auch Triamine sein oder auch Hydrazine. Als Beispiele solcher Amine seien genannt: primäre Monoamine wie Methylamin, Ethylamin, Propylamin, Isopropylamin, n-Butylamin, 2-Aminobutan, n-Dodecylamin, Stearylamin, Cyclohexylamin, Hexylamin, 2-Ethylhexylamin, 2-Propenylamin, 2-Aminoethanol, 2-Methoxyethylamin, 3-Methoxypropylamin, 3-Ethoxypropylamin, 3-Aminopropanol-(1), 1-Aminopropanol-(2), Anilin, 2-Chloranilin, 3-Chloranilin, 4-Chloranilin, 3-Nitroanilin, 2-Cyananilin, 3-Cyananilin, 4-Cyananilin, 3,4-Dichloranilin, 2-Aminophenol, 3-Aminophenol, 4-Aminophenol, Benzylamin, 4-Fluor-3-chlor-anilin, 4-Fluoranilin, 2,6-Diethylanilin, Naphthylamin, 4-Aminobenzoesäure, 2-Aminobenzoesäure, 3-Aminobenzoesäure, 2-Aminobenzolsulfonsäure, 4-Aminobenzolsulfonsäure, 3-Aminobenzolsulfonsäure, Aminotriazol, Aminotriazin, 2-Aminothiadiazol, Aminopyridin, Aminobenzthiazol, Aminooxthiazol, Aminooxdiazol, 5-Amino-5-chloro-2-phenyl-3-(2H)-pyridazinon, Aminopyridin-carbonsäure;

primäre Diamine wie Ethylendiamin, 1,2-Diaminopropan, 1,4-Diaminobutan, 1,6-Diaminohexan, 2,5-Diamino-2,5-dimethylhexan, 2-N-(2-Aminoethyl)-amino-ethanol, 2-N-Methyl-N-(3-Aminopropyl)-aminoethanol, 1,4-Bis-(3-amino-

propoxy)-butan, N,N-Bis-(3-aminopropyl)-methylamin, Tripropylentriamin; primäre Triamine wie Tripropylentetraamin.

Als Hydrazine seien beispielsweise genannt: Hydrazin, Methylhydrazin, Ethylhydrazin, N,N-Dimethylhydrazin, N,N-Diethylhydrazin, (2-Hydroxypropyl)-hydrazin, (4-Hydroxybutyl)-hydrazin, N,N-Bis-(2-hydroxyethyl)-hydrazin, Semicarbazid, Thiosemicarbazid, 1-Aminopiperidin, (2-Cyanethyl)-hydrazin, N-Amino-α-pyridon, N-Methyl-N-phenyl-hydrazin, 4-Fluorphenylhydrazin, 2-Chlorphenylhydrazin, 4-Chlorphenylhydrazin, 2-Nitrophenyl-hydrazin, 2-Methoxyphenyl-hydrazin, N,N-Diphenylhydrazin.

Die erfindungsgemäße Aminolyse wird bei Temperaturen von 0 bis 200°C, vorzugsweise von 50 bis 180°C vorgenommen. Besonders bevorzugt sind bei Verwendung aliphatischer Amine Temperaturen von 80 bis 110°C und bei der Verwendung von aromatischen Aminen und Hydrazinen Temperaturen von 110 bis 160°C.

Das erfindungsgemäße Verfahren wird im allgemeinen bei Normaldruck durchgeführt. Es ist jedoch auch möglich, das erfindungsgemäße Verfahren bei einem Unter- bzw. Überdruck (beispielsweise im Druckbereich von 0,1 bis 10 bar) durchzuführen.

Bei dem erfindungsgemäßen Verfahren werden die Reaktanden im allgemeinen in etwa äquivalenten Mengen - bezogen auf primäre Aminogruppen und zu aminolysierende Acylgruppen - eingesetzt. Es ist jedoch auch möglich, einen der Reaktanden, z.B. das Amin, im Überschuß einzusetzen.

Das erfindungsgemäße Verfahren kann sowohl ohne als auch in inerten Lösungsmitteln durchgeführt werden. Bevorzugt ist die Durchführung des erfindungsgemäßen Verfahrens in Gegenwart von inerten Lösungsmitteln.

Unter inerten Lösungsmitteln für das erfindungsgemäße Verfahren versteht man solche, die sich unter den Reaktionsbedingungen nicht verändern. Als Lösungsmittel seien beispielsweise genannt: Alkane wie Petrolether, Waschbenzin, Leichtbenzin, Hexan, Cyclohexan oder Decan, aromatische Kohlenwasserstoffe wie Benzol, Toluol oder Ethylbenzol, halogenierte Kohlenwasserstoffe wie Methylenchlorid, Chloroform, Chlorbenzol oder 1,2-Dichlorbenzol oder Ether wie Diethylether, Dibutylether, Tetrahydrofuran oder Dioxan.

Das erfindungsgemäße Verfahren kann beispielsweise wie folgt durchgeführt werden:

Die Reaktanden werden, gegebenenfalls in Anwesenheit eines geeigneten Lösungsmittels, miteinander gemischt und anschließend bei der gewünschten Umsetzungstemperatur bis zur Beendigung der Reaktion gerührt; man kann aber auch eine Reaktionskomponente vorlegen und die andere Komponente bei der gewünschten Umsetzungstemperatur, gegebenenfalls in Form einer Schmelze oder

einer Lösung in einem geeigneten Lösungsmittel oder gasförmig langsam zudosieren.

Die bei der erfindungsgemäßen Umsetzung anfallenden Reaktionsprodukte können, gegebenenfalls nach Entfernung des Lösungsmittels, von den als Nebenprodukt entstehenden Carbonsäureamiden nach bekannten Verfahren wie Destillation, Sublimation oder Kristallisation aus geeigneten Lösungsmitteln abgetrennt werden.

Die erfindungsgemäß herstellbaren N,N'-disubstituierten 5-Imino-imidazolidindion-Derivate sind außerordentlich thermostabil; selbst durch Erhitzen auf Temperaturen um 300° werden sie nicht gespalten.

Die erfindungsgemäßen N,N'-disubstituierten 5-Imino-imidazolidindion-Derivate sind hochwertige Katalysatoren für Polyisocyanat-Additionsreaktionen, z.B. für die Herstellung von Polyurethan-Schäumen.

Die erfindungsgemäß erhältlichen 5-Acylimino-imidazolidindione der Formel (I), in der x 1 ist, sind neu. Diese neuen 5-Hydrazonoimidazolidindione sind jedoch instabil und lagern sich bereits während ihrer Bildung in isomere 5-Iminoimidazolidinon-Derivate, nämlich 4-Hydroxy-5-azo-imidazolin-2-one, um.

0158921

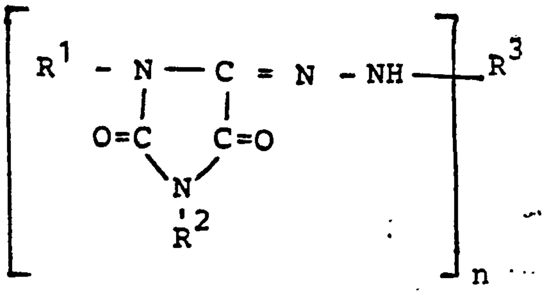

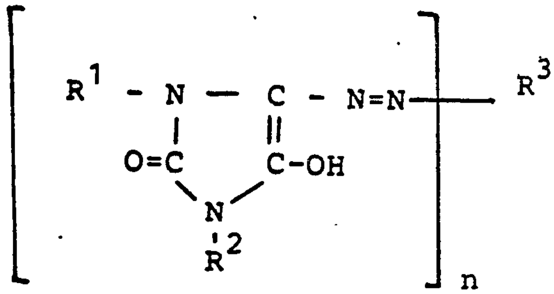

(IV) (V)

instabil stabil

Die Erfindung betrifft daher nicht nur ein neues Verfahren zur Herstellung von N,N'-disubstituierten 5-Imino-imidazolindionen der Formel (I) sondern auch neue Derivate dieser N,N'-disubstituierten 5-Imino-imidazolidindione der Formel

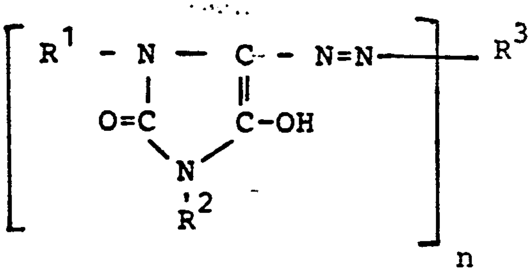

(V)

in der

$R^1$,$R^2$, $R^3$ und n die unter Formel (I) angegebene Bedeutung haben.

Als Beispiele dieser Derivate seien genannt:
4-Hydroxy-5-phenylazo-N,N'-dimethylimidazolin-2-on,
4-Hydroxy-5-phenylazo-N,N'-diethylimidazolin-2-on,
4-Hydroxy-5-phenylazo-N,N'-diphenylimidazolin-2-on,
4-Hydroxy-5-ethylazo-N,N'-dimethylimidazolin-2-on,

4-Hydroxy-5-butylazo-N,N'-dimethylimidazolin-2-on,
4-Hydroxy-5-(o-chlorphenyl)-azo-N,N'-dimethylimidazolin-2-on,
4-Hydroxy-5-(o-chlorphenyl)azo-N,N'-diphenylimidazolin-2-on,
4-Hydroxy-5-(p-chlorphenyl)azo-N,N'-dimethylimidazolin-2-on.

Die neuen Verbindungen haben herbizide und fungizide Eigenschaften.

Beispiel 1

53,4 g (0,2 Mol) 5-Acetylimino-N,N'-dibutylimidazolidin-dion werden bei 80°C in 100 ml Toluol gelöst. Die Lösung wird innerhalb von 30 Minuten tropfenweise mit 14,6 g (0,2 Mol) n-Butylamin versetzt. Die Reaktionsmischung wird 4 Stunden bei 110°C gerührt. Nach dem Abkühlen auf 70°C wird vom abgeschiedenen Acetamid (8,74 g = 74,1 % der Theorie) abgesaugt und die flüssige Phase im Vakuum eingeengt. Der Rückstand wird in 200 ml kalten Petrolether aufgenommen. Nach dem Abfiltrieren vom Ungelösten wird die flüssige Phase im Vakuum vom Lösungsmittel befreit. Der Rückstand wird destilliert. Es werden 54,3 g (96,7 % der Theorie) 5-Butylimino-N,N'-dibutylimidazolidindion erhalten.
Siedepunkt: 130°C/0,05 Torr.

Beispiel 2

98,7 g (0,3 Mol) 5-Benzoylimino-N,N'-dibutylimidazolidin-dion werden in 80 ml Toluol gelöst. Die Lösung wird auf 80°C erwärmt und innerhalb von 10 Minuten mit 21,9 g (0,3 Mol) n-Butylamin versetzt. Die Reaktionsmischung wird 3 Stunden bei 120°C gerührt und dann auf Raumtemperatur abgekühlt. Das abgeschiedene Benzamid (34,8 g = 95,9 % der Theorie) wird abgetrennt. Die flüssige Phase wird im Vakuum eingeengt und der Rückstand destilliert. Das Destillat wird in 200 ml Leichtbenzin aufgenommen. Nach dem Abtrennen der nicht gelösten Feststoffe wird die Lösung im Vakuum vom Lösungsmittel befreit. Es werden 77,1 g (91,5 % der Theorie)

5-Butylimino-N,N'-dibutylimidazolidindion erhalten.

Beispiel 3

53,4 g (0,2 Mol) 5-Acetylimino-N,N'-dibutylimidazolidindion werden in 100 ml Toluol gelöst. Die Lösung wird mit 11,6 g (0,1 Mol) 1,6-Diaminohexan versetzt und 3 1/2 Stunden bei 80°C gerührt. Das Reaktionsgemisch wird im Vakuum vom Lösungsmittel befreit. Der Rückstand wird in Leichtbenzin aufgenommen, das ungelöste Acetamid abfiltriert und die flüssige Phase abermals im Vakuum eingeengt. Es bleibt ein Öl zurück, das nach 2 Tagen kristallisiert. Es werden 37,8 g (71,0 % der Theorie) 1,6-Bis-(5-imino-N,N'-dibutylimidazolidindion)-n-hexan erhalten.
Fp.: 58°C.

Beispiel 4

53,4 g (0,2 Mol) 5-Acetylimino-N,N'-dibutylimidazolidindion werden in 100 ml Toluol gelöst. Die Lösung wird mit 6 g (0,1 Mol) Ethylendiamin versetzt und 2 1/2 Stunden bei 80°C gerührt. Das Reaktionsgemisch wird im Vakuum vom Lösungsmittel befreit. Der Rückstand wird in Leichtbenzin erhitzt, der feste Rückstand von der flüssigen Phase getrennt und die flüssige Phase abgekühlt. Das 1,2-Bis-(5-imino-N,N'-dibutylimidazolidindion)-ethan scheidet sich in Form farbloser Kristalle ab.
Ausbeute: 19,5 g (41,0 % der Theorie)
Fp.: 72°C.

Beispiel 5

45,8 g (0,25 Mol) 5-Acetylimino-N,N'-dimethylimidazolidindion werden in 250 ml Toluol mit 67,3 g (0,25 Mol) Stearylamin 8 Stunden am Rückfluß gekocht. Nach Kühlen auf Raumtemperatur wird abgesaugt, einrotiert und zweimal aus je 250 ml Methanol umkristallisiert.
Ausbeute: 86,3 g (87,8 % der Theorie) 5-Stearylimino-N,N'-dimethylimidazolidindion
Schmp.: 64°C.

Beispiel 6

70,5 g (0,264 Mol) 5-Acetylimino-N,N'-dibutylimidazolidindion werden mit 26,9 g (0,264 Mol) 1-Amino-3-dimethylaminopropan in 100 ml Toluol 8 Stunden bei 110°C gerührt, auf Raumtemperatur gekühlt, vom Unlöslichen abgesaugt, einrotiert, in 200 ml Petrolether aufgekocht, auf 5°C gekühlt, vom Niederschlag abgesaugt, einrotiert und im Vakuum destilliert.
Siedepunkt: 135 bis 140°C/0,1 Torr.
Ausbeute: 67,2 g (82,1 % der Theorie) 5-(3-Dimethylaminopropyl)-imino-N,N'-dibutylimidazolidindion
Gelbe Flüssigkeit

Beispiel 7

33,6 g (0,126 Mol) 5-Acetylimino-N,N'-dibutylimidazolidindion werden mit 33,8 g (0,126 Mol) Stearylamin in 100 ml Toluol 8 Stunden bei 110°C gerührt. Nach Kühlen

auf Raumtemperatur wird abgesaugt, einrotiert, in 200 ml Petrolether aufgekocht, auf 5°C gekühlt, abgesaugt, einrotiert und im Vakuum destilliert.
Siedepunkt: 250°C/0,05 Torr.
Ausbeute: 37,6 g (62,6 % der Theorie) 5-Stearylimino-N,N'-dibutylimidazolidindion
Gelbes Öl, das bei Raumtemperatur erstarrt.
Schmp.: 28 bis 30°C

Beispiel 8

36,6 g (0,2 Mol) 5-Acetylimino-imidazolidindion werden in 100 ml Toluol mit 37,0 g (0,2 Mol) Dodecylamin gemischt und 4 Stunden bei 110°C gerührt. Nach Einrotieren wird in Petrolether aufgekocht, auf Raumtemperatur gekühlt, abgesaugt und einrotiert. Der Rückstand wird in Ethanol umkristallisiert.
Schmp.: 47°C farbloser Feststoff
Ausbeute: 41,7 g (67,5 % der Theorie) 5-Dodecylimino-N,N'-dimethylimidazolidindion

Beispiel 9

53,4 g (0,2 Mol) 5-Acetylimino-N,N'-dibutylimidazolidindion werden in 100 ml Toluol mit 18,6 g (0,2 Mol) Anilin gemischt und 3 Stunden bei 110°C gerührt. Nach Kühlen auf Raumtemperatur wird abgesaugt, der Feststoff in 300 ml Wasser aufgekocht, kalt abgesaugt und getrocknet.

Farbloser Feststoff
Ausbeute: 36,7 g (61 % der Theorie)
Schmp.: 151°C

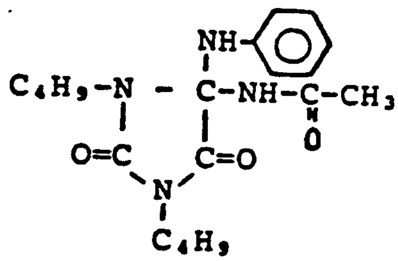

36,0 g (0,1 Mol) des Adduktes werden in 100 ml o-Dichlorbenzol 8 Stunden bei 160°C gerührt. Nach Kühlen auf Raumtemperatur wird einrotiert, der Rückstand in 100 ml $H_2O$ suspendiert, in 100 ml Methylenchlorid aufgenommen, über $Na_2SO_4$ getrocknet, abfiltriert, einrotiert und destilliert.
Siedepunkt: 165°C/0,05 Torr
Ausbeute: 24,8 g (82,4 % der Theorie) 5-Phenyl-imino-N,N'-dibutylimidazolidindion in Form eines gelben Öles.

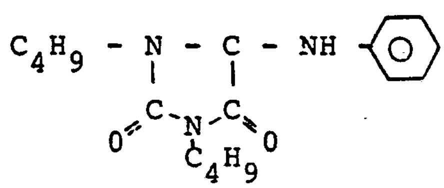

| | C | H | N |
|---|---|---|---|
| ber.: | 67,8 | 7,6 | 14,0 |
| gef.: | 67,7 | 7,6 | 13,9 |

Beispiel 10

39,33 g (0,215 Mol) 5-Acetylimino-N,N'-dimethylimidazolidindion werden in 100 ml Toluol mit 30,3 g (0,213 Mol)

2-Chlorphenylhydrazin 8 Stunden bei 110°C gerührt. Es wird heiß abgesaugt, der Feststoff in 300 ml Wasser bei 20°C 1 Stunde gerührt, abgesaugt und der Feststoff aus 200 ml Ethanol/Wasser 1:1 umkristallisiert.
Ausbeute: 18,3 g (32,2 % der Theorie) 4-Hydroxy-5-(o-chlorphenyl)azo-N,N'-dimethyl-4-imidazolin-2-on in Form eines gelben Feststoffes
Schmp.: 216°C

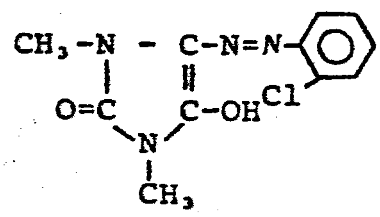

Beispiel 11

267 g (1 Mol) 5-Acetylimino-N,N'-dibutylimidazolidindion, 103 g (1 Mol) Phenylhydrazin und 500 ml 1,2-Dichlorbenzol werden gemischt und 8 Stunden bei 170°C gerührt. Nach Kühlen auf 20°C wird abgesaugt, das 1,2-Dichlorbenzol bei 15 mbar abdestilliert, der Feststoff 1 Stunde bei 20°C in 500 ml Wasser verrührt, abgesaugt und über Phosphorpentoxid getrocknet.
Ausbeute: 219 g (69,3 % der Theorie) 4-Hydroxy-5-(phenyl)azo-N,N'-dibutyl-4-imidazolin-2-on in Form eines gelben Feststoffes
Schmp.: 149°C

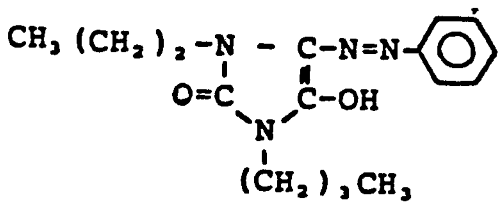

Beispiel 12

Anwendungsbeispiel 4-Hydroxy-5-(o-chlorphenyl)-azo-N,N'-dimethyl4-imidazolin-2-on

Die Fungizide Wirksamkeit von 4-Hydroxy-5-(o-chlorphenyl)azo-N,N'-dimethyl-4-imidazolin-2-on wurde in einem Gewächshaustest geprüft.

Wirkung gegen

Pyricularia oryzae: 30 %
Phytophthora infestans: 80 %

(0 % = keine Wirkung; 100 % = Befallsfreiheit).

Patentansprüche

1) Verfahren zur Herstellung von N,N'-disubstituierten 5-Imino-imidazolidindionen, dadurch gekennzeichnet, daß man aus N,N'-disubstituierten 5-Acylimino-imidazolidindionen den Acylrest durch Aminolyse mit primären Aminen abspaltet.

2) Verfahren nach Anspruch 1, dadurch gekennzeichnet, daß man die Aminolyse bei Temperaturen von 0 bis 200°C vornimmt.

3) Verfahren nach Anspruch 1 und/oder 2, dadurch gekennzeichnet, daß man N,N'-disubstituierte 5-Acylimino-imidazolidindione und primäre Amine in etwa äquivalenten Mengen, bezogen auf zu aminolysierende Acylgruppen und primäre Aminogruppen, einsetzt.

4) Verfahren nach Anspruch 1, 2 und/oder 3, dadurch gekennzeichnet, daß man die Aminolyse in Gegenwart eines inerten Lösungsmittels durchführt.

5) Verfahren nach einem der Ansprüche 1 bis 4, dadurch gekennzeichnet, daß man es zur Herstellung von N,N'-disubsituierten 5-Imino-imidazolindionen der Formel

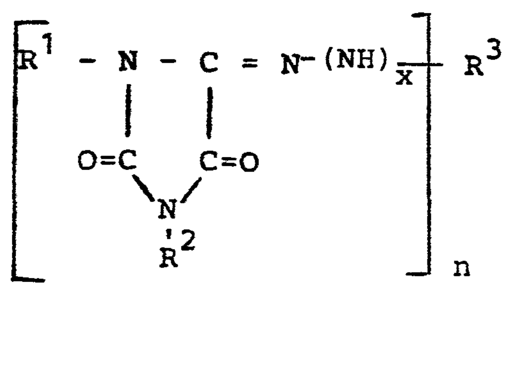

verwendet, in der

n 1, 2 oder 3 bedeutet,

x 0 oder 1 ist,

$R^1$ und $R^2$ unabhängig voneinander für einen gegebenenfalls substituierten aliphatischen, cycloaliphatischen, araliphatischen, aromatischen oder heteroaromatischen Rest stehen und

$R^3$ für einen n-wertigen, gegebenenfalls substituierten aliphatischen, cycloaliphatischen, aromatischen oder heteroaromatischen Kohlenwasserstoff-Rest steht,

und als Ausgangsverbindungen primäre Amine der Formel

$$[H_2N-(NH)_x]_n-R^3$$

in der n, x und $R^3$ die vorstehend angegebene Bedeutung haben, und 5-Acylimino-imidazolidindione der Formel

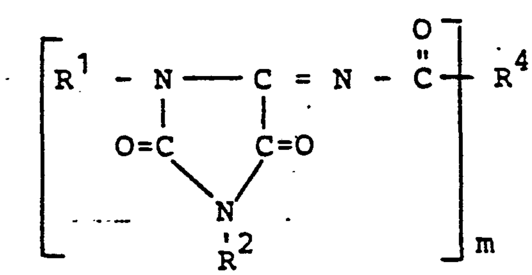

verwendet, in der

m 1, 2 oder 3 bedeutet,

$R^1$ und $R^2$ die vorstehend angegebene Bedeutung haben, und

$R^4$ für einen m-wertigen gegebenenfalls substituierten aliphatischen, cycloaliphatischen, araliphatischen oder aromatischen Rest steht.

6) 4-Hydroxy-5-azo-imidazolin-2-one der Formel

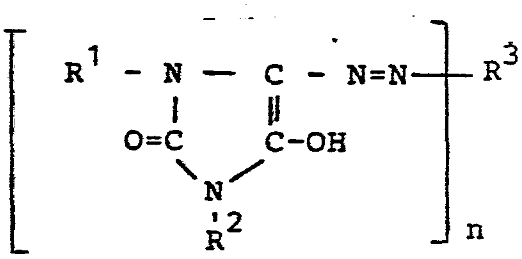

in der

n 1, 2 oder 3 bedeutet,

$R^1$ und $R^2$ unabhängig voneinander für einen gegebenenfalls substituierten aliphatischen, cycloaliphatischen, araliphatischen, aromatischen oder heteroaromatischen Rest stehen und

$R^3$ für einen n-wertigen, gegebenenfalls substituierten aliphatischen, cycloaliphatischen, aromatischen oder heteroaromatischen Kohlenwasserstoff-Rest steht.

7) 4-Hydroxy-5-azo-imidazolin-2-one gemäß Anspruch 6, dadurch gekennzeichnet, daß in der in Anspruch 6 angegebenen Formel n = 1 ist.